(19) Europäisches Patentamt
European Patent Office
Office européen des brevets

(11) EP 2 816 030 A1

(12) EUROPEAN PATENT APPLICATION

(43) Date of publication:
**24.12.2014 Bulletin 2014/52**

(51) Int Cl.:
***C07D 209/88*** *(2006.01)*

(21) Application number: **13382233.8**

(22) Date of filing: **20.06.2013**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **Duke Chem, S. A.**
**08799 Olerdola Barcelona (ES)**

(72) Inventors:
- **Silva Guisasola, Octavio**
  **47162 ALDEAMAYOR DE SAN MARTÍN (ES)**
- **Esteve Casol, Judit**
  **08799 OLÈRDOLA (Barcelona) (ES)**

(74) Representative: **ZBM Patents - Zea, Barlocci & Markvardsen**
**Plaza Catalunya, 1**
**08002 Barcelona (ES)**

(54) **Process for the preparation of frovatriptan and its enantiomer**

(57) It relates to the compounds of formula [II-(R,S)] and formula [II-(S,R)], and to a process for their preparation which comprises: a) reacting an enantiomeric mixture containing a compound of formula [I-(R)] and a compound of formula [I-(S)] with a chiral agent in the presence of a solvent mixture; b) isolating the compound of formula [II-(R,S)] or, alternatively, the compound of formula [II-(S,R)] from the corresponding diastereomeric mixture; and c) optionally purifying them. It also relates to a process for the preparation of frovatriptan and its enantiomer from a compound of formula [II-(R, S)] or a compound of formula [II-(S,R)] respectively.

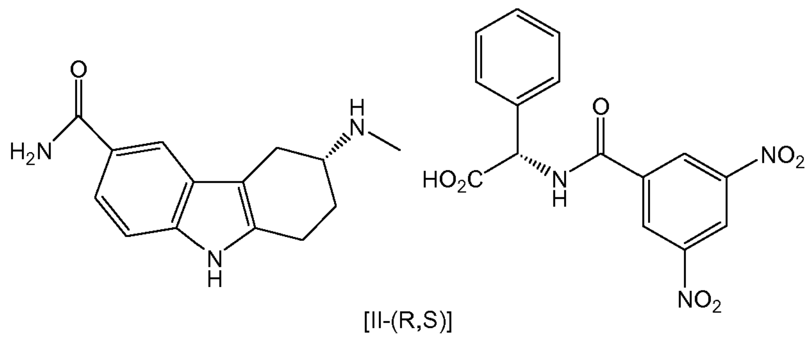

[II-(R,S)]

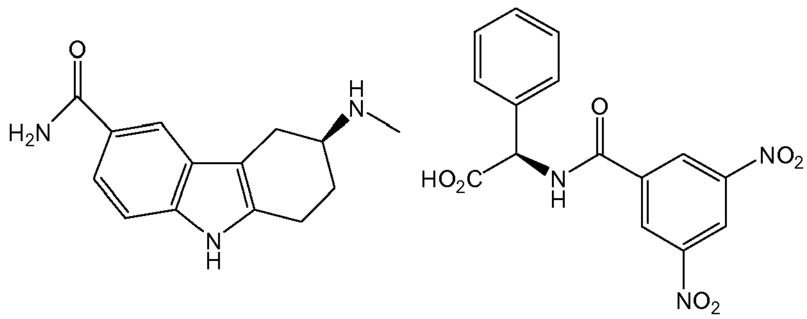

[II-(S,R)]

EP 2 816 030 A1

## Description

The present invention relates to diastereomeric salts of formula [II-(R,S)] or formula [II-(S,R)] derived from frovatriptan or its enantiomer, respectively, and one enantiomer of (3,5-dinitrobenzoyl)phenylglycine. It also relates to a process for the preparation of these diastereomeric salts and to a process for the preparation of frovatriptan or its enantiomer.

BACKGROUND ART

Frovatriptan is the generic name of compound (R)-6-carboxamido-3-N-methylamino-1,2,3,4-tetrahydrocarbazole, the chemical structure of which is the following:

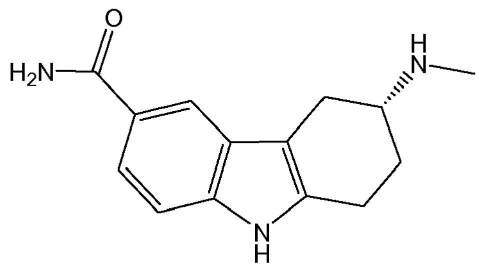

[I-(R)]

Frovatriptan is currently used for the treatment of migraine. It was first disclosed as a racemic compound in document WO 93/00086. This document describes compound of formula (I) including frovatriptan as 5HT1 receptor agonists. The preparation of frovatriptan as a racemic compound is specifically described in example 24 of this patent application. Furthermore, this document discloses that the enantiomers of the compounds of formula (I) can be separated by chiral HPLC or by formation of diastereomeric salts with optically active acids and later separation of the diastereomeric salts by crystallization. As optically active acids D-tartaric acid, L-malic acid, L-mandelic acid, L-gulonic acid or 2,3,4,6-di-O-isopropylidene-keto-L-gulonic acid are disclosed. However, this document does not disclose frovatriptan nor its enantiomer.

Document WO 94/14772 discloses specifically frovatriptan and its enantiomer and a process for their preparation by chiral resolution of the racemic compound. In particular, it discloses that the separation of the enantiomers can be carried out: (a) by chromatography (e.g. chiral HPLC): (b) by formation of diastereomeric salts which are separated by crystallization or chromatography; or (c) by alkylation and subsequent transformation into frovatriptan. As optically active acids which can be used for the formation of diastereomeric salts, this document cites (1 S)-(+)-camphor-10-sulfonic acid, D-tartaric acid, L-malic acid, L-mandelic acid, L-gulonic acid, 2,3,4,6-di-O-isopropylidene-2-keto-L-gulonic acid, and (R)-2-pyrrolidone-5-carboxylic acid (also known as D-pyroglutamic acid).

In the examples (1S)-(+)-camphor-10-sulfonic acid (example 3) and D-pyroglutamic acid (example 13) are used. However, it is disclosed that with (1S)-(+)-camphor-10-sulfonic acid ten recrystallization steps are needed to obtain the desired enantiomeric purity making this process not suitable from an industrial point of view. Additionally, the disclosed yield obtained by using D-pyroglutamic is very low (25%).

On the other hand, attempts to reproduce the process with D-pyroglutamic acid failed in the hands of the present inventors. This irreproducibility (inconsistency) in the formation of the diastereomeric salt is also described by the inventors of the patent application WO 2012/147020.

This document, WO 2012/147020, discloses a process for the chiral resolution of frovatriptan by forming a diastereomeric salt with di-p-toluoyl-(D)-(+)-tartaric acid (DPTTA). However, the enantiomeric excess described in the examples (>98%) is not acceptable to be used as an active ingredient in pharmaceutical preparations and, in any case, no yields are given. Other chiral agents such as (R)-(+)-2-(4-hydroxyphenoxy)propionic acid, (S)-(+)-2-(4-hydroxyphenoxy)propionic acid, N-acetyl-L-glutamic acid, N-acetyl-D-glutamic acid, N-CBZ-L-glutamic acid, N-BOC-L-glutamic acid, dibenzoyl-L(+)-tartaric acid, dibenzoyl-D-(+)-tartaric acid, and L-lysine are also disclosed although not exemplified.

Therefore, in view of the prior art, there is a need of having alternative processes for the chiral resolution of 6-carboxamido-3-N-methylamino-1,2,3,4-tetrahydrocarbazole to obtain frovatriptan and its enantiomer, in particular, processes which are easy to industrialize.

SUMMARY OF THE INVENTION

As mentioned above, some chiral acids have been described in the prior art for the separation of frovatriptan and its enantiomer by the formation and separation of diastereomeric salts. However, these processes either do not allow obtaining acceptable enantiomeric purities in good yields and/or make necessary several consecutive recrystallization steps in order to achieve the desired enantiomeric purity, in particular the enantiomeric purity required for an active ingredient.

The inventors have found that when in the chiral resolution process for preparing the desired enantiomer of 6-carboxamido-3-N-methylamino-1,2,3,4-tetrahydrocarbazole, one of the two enantiomers of (3,5-dinitrobenzoyl)-phenylglycine (DNBG) is used as a chiral agent, the process takes places in higher yields and higher enantiomeric purities in comparison with the known chiral agents as it will be shown in the examples.

Moreover, the process of the invention is particularly suitable for industrialization because there is no need to carry out many purification steps to achieve the desired enantiomeric purity, and as a consequence, the yields are higher. In particular, a single crystallization step allows obtaining an enantiomeric excess equal to or higher than 93%, more particularly equal to or higher than 98%, and, if additional recrystallization steps are carried out even higher enantiomeric purities can be obtained.

Therefore, a first aspect of the invention relates to a process for the preparation of either a compound of formula [II-(R,S)] or, alternatively, a compound of formula [II-(S,R)]

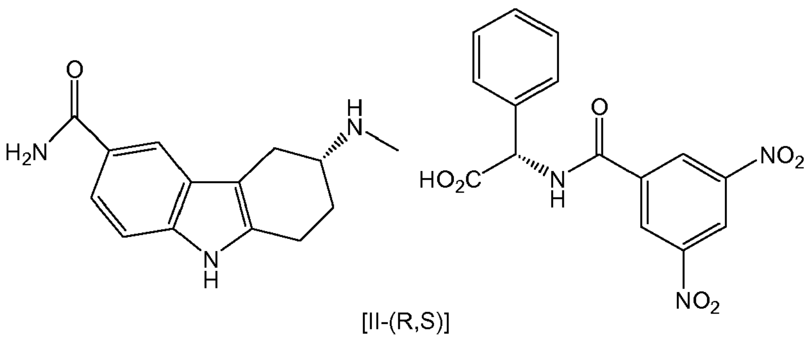

[II-(R,S)]

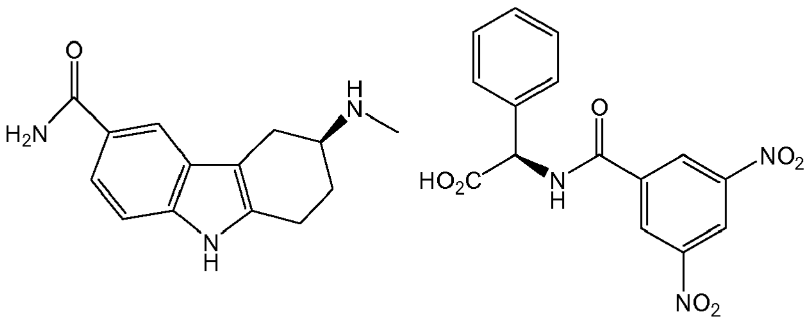

[II-(S,R)]

which comprises the following steps:

(a) reacting an enantiomeric mixture formed by the two enantiomers of 6-carboxamido-3-N-methylamino-1,2,3,4-tetrahydrocarbazole (I), which are the compound of formula [I-(R)] and the compound of formula [I-(S)]

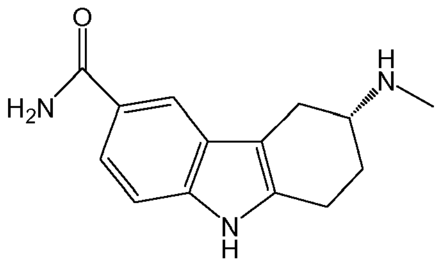

[I-(R)]

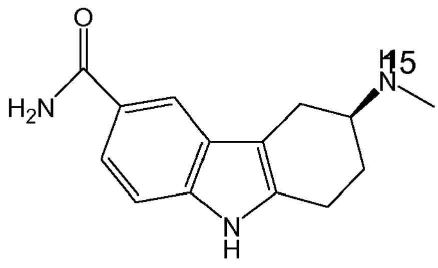

[I-(S)]

with a chiral agent selected from the two enantiomers of (3,5-dinitrobenzoyl)-phenylglycine (III) which are the compound of formula [III-(S)] and the compound of formula [III-(R)]

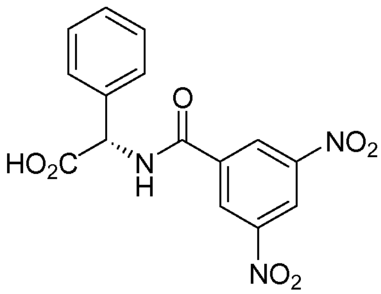

[III-(S)]

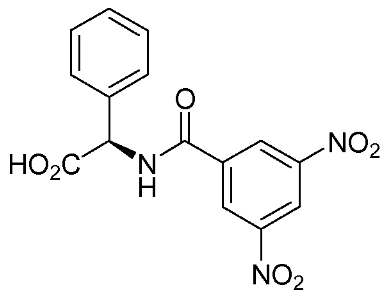

[III-(R)]

in the presence of a solvent mixture containing water and one or more organic solvents which are miscible with water; to give a diastereomeric mixture formed by one of the enantiomers of the compound of formula (I) with one of the enantiomers of the compound of formula (III), the diastereomeric mixture containing either:

A) a compound of formula [II-(R,S)] and a compound of formula [II-(S,S)] when the chiral agent used is a compound of formula [III-(S)]; or alternatively, B) a compound of formula [II-(S,R)] and a compound of formula [II-(R,R)] when the chiral agent used is a compound of formula [III-(R)];
(b) isolating either the compound of formula [II-(R,S)] or, alternatively, the compound of formula [II-(S,R)] from the corresponding diastereomeric mixture of step (a) whereby either the compound of formula [II-(S,S)], or alternatively, the compound of formula [II-(R,R)] remains in solution; and
(c) optionally purifying either the compound of formula [II-(R,S)] or, alternatively, the compound of formula [II-(S,R)] obtained in step (b).

A second aspect of the invention relates to a process for the preparation of either frovatriptan of formula [I-(R)], or its enantiomer of formula [I-(S)] which comprises:

(d) submitting either the compound of formula [II-(R,S)] or, alternatively, the compound of formula [II-(S,R)] obtained in step (c) to either a base treatment, or alternatively, to first an acid treatment and then a base treatment to give a compound of formula [I-(R)], or a compound of formula [I-(S)], respectively;

(e) isolating either the compound of formula [I-(R)] or, alternatively, the compound of formula [I-(S)] from the corresponding reaction medium of step (d); and

(f) optionally reacting either the compound of formula [I-(R)], or, alternatively, the compound of formula [I-(S)] obtained in step (d) with an acid to give the corresponding pharmaceutically acceptable salt.

Other aspects of the invention relate to a compound of formula [II-(R,S)] and to a compound of formula [II-(S,R)].

DETAILED DESCRIPTION OF THE INVENTION

The term "enantiomeric mixture" as used herein refers to a mixture of two enantiomers. In the present invention the enantiomeric mixture of a compound of formula (I) refers to a mixture containing a compound of formula [I-(R)] and a compound of formula [I-(S)] in any proportion. The term "enantiomeric mixture" may refer both to a racemic mixture and a mixture enriched in one of the two enantiomers.

The term "racemic mixture" or "racemate" as used herein refers to a mixture of enantiomers which comprises equal amounts of a compound of formula [I-(R)] and a compound of formula [I-(S)], i.e. the molar ratio of the two enantiomers in the racemic mixture is 50:50.

The term "mixture enriched in one enantiomer" as used herein refers to a mixture of enantiomers [I-(R)] and [I-(S)], which comprises a higher amount of one enantiomer ([I-(R)] or [I-(S)]) with respect to the other enantiomer ([I-(S)] or [I-(R)] respectively), i.e. the molar ratio of the two enantiomers in the enantiomerically enriched mixture is other than 50:50.

The term "diastereomeric mixture" as used herein refers to the mixture of stereoisomers having two or more chiral centers that are not enantiomers. A diastereomeric such as the mixture of a compound of formula [II-(R,S)] and a compound of formula [II-(S,S)]; or the mixture of a compound of formula [II-(S,R)] and a compound of formula [II-(R,R)] is formed when reacting an enantiomeric mixture formed by the two enantiomers of compound of formula (I), which are the compound of formula [I-(R)] and the compound of formula [I-(S)], with a chiral agent selected from the two enantiomers of (3,5-dinitrobenzoyl)-phenylglycine (III) which are the compound of formula [III-(S)] and the compound of formula [III-(R)].

The term "enantiomeric excess" (ee) refers to the difference between the amounts of each of the enantiomers present in a mixture, relative to the total amount of the compound in the mixture expressed as percentage (x 100%). For an excess of the R-enantiomer, the enantiomeric excess can be calculated by the following formula:

$$ee\ \% = ([R] - [S]) / ([R] + [S]) \times 100$$

For an excess of the S-enantiomer, the enantiomeric excess can be calculated by the following formula:

$$ee\ \% = ([S] - [R]) / ([R] + [S]) \times 100$$

The term "enantiomeric purity" (ep) refers to the purity of an enantiomer with respect to the other enantiomer. For an excess of the R-enantiomer, the enantiomeric purity can be calculated by the following formula:

$$ep\ \% = ([R] / ([R] + [S]) \times 100$$

For an excess of the S-enantiomer, the enantiomeric purity can be calculated by the following formula:

$$ep\ \% = ([S] / ([R] + [S]) \times 100$$

In the above formulas, [R] and [S] are the respective molar fractions of the enantiomers in a mixture such that [R] + [S] = 1.

The term "precipitate" as used herein refers to the process of causing solid substances, such as crystals, to be separated from a solution. The precipitation may include crystallization.

For the purposes of the invention, room temperature is 20-25 °C and reflux temperature refers to the boiling point of the solvent mixture being heated at atmospheric pressure,

The term "organic solvents miscible with water" is used herein to refer to an organic solvent or mixture thereof which is capable of mixing with water forming a homogeneous solution, i.e., without forming a separation of phases, at least in a particular ratio and at least at a given temperature, preferably at least at a temperature comprised from room temperature to the reflux temperature of the solvent mixture, more preferably at least at room temperature.

As mentioned above, a first aspect of the invention relates to a process for the preparation of a compound of formula [II-(R,S)] or, alternatively, a compound of formula [II-(S,R)] from an enantiomeric mixture containing both frovatriptan ((R)-(+)-6-carboxamido-3-N-methylamino-1,2,3,4-tetrahydrocarbazole) of formula [I-(R)], and its enantiomer ((S)-(+)-6-carboxamido-3-N-methylamino-1,2,3,4-tetrahydrocarbazole) of formula [I-(S)].

The first step of the process (step (a)) comprises reacting the enantiomeric mixture containing both [I-(R)] and [I-(S)] with an optically active acid (also referred herein to as chiral agent) which is one of the enantiomers of (3,5-dinitrobenzoyl)phenylglycine (DNBG) in the presence of a solvent mixture containing water and one or more organic solvents which are miscible with water.

When the chiral agent is (S)-(3,5-dinitrobenzoyl)phenylglycine ((S)-DNBG) of formula [III-(S)], the obtained diastereomeric salt mixture contains the compound of formula [II-(R,S)] (i.e. the salt resulting from frovatriptan [I-(R)] and the acid [III-(S)]); and the compound of formula [II-(S,S)] (i.e. the salt resulting from the enantiomer of frovatriptan [I-(S)] and the acid [III-(S)]).

When the chiral agent is (R)-(3,5-dinitrobenzoyl)phenylglycine ((R)-DNBG) of formula [III-(R)], the obtained diastereomeric salt mixture contains the compound of formula [II-(S,R)] (i.e. the salt resulting from the enantiomer of frovatriptan [I-(S)] and the acid [III-(R)]); and a compound of formula [II-(R,R)] (i.e. the salt resulting from frovatriptan [I-(R)] and the acid [III-(R)]).

The diastereomeric salts of formula [II-(R,S)], [II-(S,S)], [II-(S,R)] and [II-(R,R)] also form part of the invention. In a preferred embodiment, the invention relates to the diastereomeric salts of formula [II-(R,S)] and [II-(S,R)], more preferably to the diastereomeric salt of formula [II-(R,S)].

The pair of compounds [II-(R,S)] and [II-(S,S)], as well as [II-(S,R)] and [II-(R,R)], are diastereomers of each other, and show different physical properties, such as solubility and crystallization characteristics, which allow their separation from one another. In particular, when these salts are formed in the presence of a solvent mixture containing water and one or more organic solvents miscible in water, mainly either the salt [II-(R,S)] or, alternatively, the salt [II-(S,R)] precipitates from the corresponding reaction medium, whereas the salts of the other enantiomer, which are [II-(S,S)] or [II-(R,R)], respectively, remain in solution.

The resolution process of the invention is carried out in the presence of a solvent mixture containing water and one or more organic solvents which are miscible with water.

Examples of organic solvents which are miscible with water at least in a particular ratio at a given temperature and may be used in step (a) include, without limitation, methanol (MeOH), ethanol (EtOH), propanol (PrOH), isopropanol ($^i$PrOH), isobutanol ($^i$BuOH), acetone, acetonitrile (ACN), tetrahydrofuran (THF), dimethylformamide (DMF), dimethylsulfoxide (DMSO), dimethylacetamide (DMA), or mixtures thereof.

In a preferred embodiment, optionally in combination with one or more features of the various embodiments described above or below, the solvent mixture contains water and an organic solvent selected from dimethylformamide (DMF), dimethylsulfoxide (DMSO), dimethylacetamide (DMA), and mixtures thereof. More preferably, the solvent mixture contains water and dimethylformamide (DMF).

The inventors have found that the amount of water present in the solvent mixture used for the resolution can be optimized to obtain the highest enantiomeric purity and yield for each given organic solvent or mixture of organic solvents. The particularly preferred amount of water to be used for each given organic solvent or mixture of organic solvents can be determined by carrying out the process of the invention at different water-organic solvent ratios and determining the obtained enantiomeric purity in each case from the teachings of the examples of the invention.

In a particular embodiment, optionally in combination with one or more features of the various embodiments described above or below, the solvent mixture contains water and an organic solvent in a volume ratio organic solvent:water comprised from 9:1 to 1:9, more preferably from 1:1 to 1:9.

In another particular embodiment, optionally in combination with one or more features of the various embodiments described above or below, the solvent mixture contains water and an organic solvent selected from isopropanol ($^i$PrOH), isobutanol ($^i$BuOH), acetone, acetonitrile (ACN), tetrahydrofuran (THF), in a volume ratio organic solvent:water comprised from 9:1 to 1:9, more preferably from 1:1 to 1:9, more preferably 7:3.

In a preferred embodiment, optionally in combination with one or more features of the various embodiments described above or below, the solvent mixture contains water and an organic solvent selected from dimethylformamide (DMF), dimethylsulfoxide (DMSO) and dimethylacetamide (DMA) in a volume ratio organic solvent:water comprised from 9:1 to 1:8, more preferably from 1:1 to 2:8, more preferably 1:2.

In another particular embodiment, optionally in combination with one or more features of the various embodiments described above or below, the molar ratio of the chiral agent and the enantiomeric mixture of [I-(R)] and [I-(S)] of step (a), particularly the racemic mixture, is from 0.4:1 to 1.6:1, more particularly from 0.5:1 to 1:1 and even more particularly 0.8:1.

In another particular embodiment, optionally in combination with one or more features of the various embodiments described above or below, step (a) is carried out at a temperature comprised from room temperature to the reflux temperature of the solvent mixture, more particularly at the reflux temperature of the solvent mixture.

In another particular embodiment, optionally in combination with one or more features of the various embodiments described above or below, the reaction is carried out by firstly heating the reaction medium as described above until total dissolution of the compounds, and later cooling the reaction medium to a temperature comprised from 0-70 °C, more particularly from 0-25 °C, even more particularly at room temperature.

As mentioned above, in the process of the present invention, mainly only either the salts [II-(R,S)] or, alternatively, [II-(S,R)] precipitate from the reaction medium, whereas the salts [II-(S,S)] or [II-(R,R)], respectively, remain in solution so that they can be easily separated from their corresponding diastereomers.

Thus, the second step of the process (step (b)) comprises isolating the compound of formula [II-(R,S)] or, alternatively, the compound of formula [II-(S,R)] from the corresponding diastereomeric mixture of step (a). The isolation of the product from the reaction medium may be carried out by several methods know in the art, such as filtration or centrifugation and may also comprise an optional drying step. In a particular embodiment, the separation is carried out by filtration.

The isolated compound of formula [II-(R,S)] or, alternatively, the compound of formula [II-(S,R)] may be optionally purified by any of the methods well-known in the art. In a particular embodiment, either the compound of formula [II-(R,S)] or, alternatively, the compound of formula [II-(S,R)] is recrystallized in an appropriate solvent mixture, such as the one used in the resolution step.

Recrystallization may be generally performed by firstly heating the compound of formula [II-(R,S)] or, alternatively, the compound of formula [II-(S,R)] in the presence of a solvent at a temperature comprised from 15 °C to the reflux temperature of the solvent mixture, more particularly under reflux, and later cooling the reaction medium to a temperature comprised from 0-70 °C, more particularly from 0-25 °C, even more particularly at room temperature, whereby the product crystallizes, isolating the product, for example by filtration and optionally drying it.

The inventors have found that when the enantiomeric purity of the diastereomeric salts of formula [II-(R,S)] and [II-(S,R)] increases, their solubility drastically decreases, so that their further purification, in particular by recrystallization, become more difficult. Thus, in a preferred embodiment, optionally in combination with one or more features of the various embodiments described above or below, the process of the invention comprises one recrystallization step in a suitable solvent. In the present invention, a single crystallization step allows obtaining an enantiomeric excess equal to or equal to or higher than 93%, more particularly equal to or higher than 98%.

If desired, one or more additional purification steps, more particularly recrystallization steps as defined above, may be performed in order to even increase the enantiomeric purity. More preferably, the recrystallization step is carried out in a solvent mixture containing water and one or more organic solvents having a high solubilizing capacity, such as polar organic solvents having a high boiling point at, preferably having a boiling point comprised from 140-200 °C). As used herein, the term "polar organic solvent" refers to an organic solvent that has a permanent electrical dipole moment. Examples of such polar solvents having a high boiling point include, without limitation, dimethylformamide (DMF), dimethylsulfoxide (DMSO), dimethylacetamide (DMA), and mixtures thereof.

In a particular embodiment, optionally in combination with one or more features of the various embodiments described above or below, the solvent mixture in which the recrystallization steps is carried out contains water and an organic solvent in a volume ratio organic solvent:water comprised from 9:1 to 1:9, more preferably from 1:1 to 1:9.

In a more preferred embodiment the solvent mixture used in the recrystallization step is the same as the solvent used for the resolution step and more preferably contains water and an organic solvent selected from dimethylformamide (DMF), dimethylsulfoxide (DMSO), dimethylacetamide (DMA), and mixtures thereof, and even more preferably in a volume ratio organic solvent:water comprised from 9:1 to 1:8, more preferably from 1:1 to 2:8, more preferably 1:2. The use of the same solvent mixture in the resolution and recrystallization steps is particularly advantageous for an industrial process since it avoids having mixtures of solvents which require intermediate drying steps.

In a preferred embodiment, optionally in combination with one or more features of the various embodiments described above or below, the invention relates to a process for the preparation of a compound of formula [II-(R,S)] which comprises the following steps:

(a') reacting an enantiomeric mixture containing a compound of formula [I-(R)] and a compound of formula [I-(S)] with a compound of formula [III-(S)] in the presence of a solvent mixture containing water and one or more organic solvents which are miscible with water; to give a diastereomeric mixture containing a compound of formula [II-(R,S)] and a compound of formula [II-(S,S)];
wherein:

> the solvent mixture preferably contains water and an organic solvent selected from dimethylformamide (DMF), dimethylsulfoxide (DMSO),
> dimethylacetamide (DMA), and mixtures thereof; and/or
> the reaction is preferably carried out at a temperature comprised from room temperature to the reflux temperature of the solvent mixture, more particularly at the reflux temperature of the solvent mixture; and/or the molar ratio of the chiral agent and the enantiomeric mixture of [I-(R)] and [I-(S)] is preferably comprised from 0.4:1 to 1.6:1; more preferably from 0.5:1 to 1:1; and

(b') isolating the compound of formula [II-(R,S)] from the diastereomeric mixture of step (a'); whereby the compound

of formula [II-(S,S)] remains in solution; wherein the isolation of the compound of formula [II-(R,S)] is preferably carried out by filtration; and

(c') optionally purifying the compound of formula [II-(R,S)] obtained in step (b'); wherein preferably at least one crystallization step is performed in a solvent mixture which preferably contains water and an organic solvent selected from dimethylformamide (DMF), dimethylsulfoxide (DMSO), dimethylacetamide (DMA), and mixtures thereof.

In a particular embodiment, optionally in combination with one or more features of the various embodiments described above or below, either the compound of formula [II-(R,S)] or, alternatively, the compound of formula [II-(S,R)] is converted into frovatriptan of formula [I-(R)], or its enantiomer of formula [I-(S)], respectively.

Thus, in a particular embodiment, the process of the invention further comprises the following steps:

(d) submitting either the compound of formula [II-(R,S)] or, alternatively, the compound of formula [II-(S,R)] to either a base treatment, or alternatively, to first an acid treatment and then a base treatment to give a compound of formula [I-(R)], or a compound of formula [I-(S)], respectively;
(e) isolating the compound of formula [I-(R)] or, alternatively, the compound of formula [I-(S)] from the reaction medium of step (d); and
(f) optionally reacting either the compound of formula [I-(R)], or, alternatively, the compound of formula [I-(S)] obtained in step (d) with an acid to give the corresponding pharmaceutically acceptable salt.

It also forms part of the invention a process for the preparation of either frovatriptan of formula [I-(R)], or, alternatively, its enantiomer of formula [I-(S)] from a compound of formula [II-(R,S)] or a compound of formula [II-(S,R)], respectively, comprising the above defined steps (d)-(f).

In a preferred embodiment, optionally in combination with one or more features of the various embodiments described above or below, the invention relates to a process for the preparation of a compound of formula [I-(R)].

Step (d) may be performed with a base treatment or with an acid-base treatment, in the presence of a solvent mixture containing water and one or more organic solvents which are not miscible with water, such as ethyl acetate, so that a separation of an aqueous phase and an organic phase takes place. Examples of bases include, without limitation, sodium hydroxide, potassium hydroxide, sodium carbonate, potassium carbonate and the like. Examples of acids include, without limitation hydrochloric acid.

If a base is used in step (d) either the compound of formula [I-(R)], or, alternatively, the compound of formula [I-(S)] remains in the organic phase and may be isolated by separating the aqueous phase (which contains the chiral agent), removing the solvent of the organic phase and optionally drying. If an acid is firstly used in step (d) the compound of formula [I-(R)], or, alternatively, the compound of formula [I-(S)] remains in the aqueous phase in salt form. After separating the organic phase containing the chiral agent, the compound may be directly isolated by removing the solvent to give either the compound of formula [I-(R)], or, alternatively, the compound of formula [I-(S)] in salt form, or may be treated with a base and later on extracted with an organic solvent such as isobutanol to give either the compound of formula [I-(R)], or, alternatively, the compound of formula [I-(S)].

In a particular embodiment, optionally in combination with one or more features of the various embodiments described above or below, step (d) is carried out with a base.

In a preferred embodiment, optionally in combination with one or more features of the various embodiments described above or below, step (d) is carried out by an acid treatment followed by a base treatment.

Additionally, either the compound of formula [I-(R)], or, alternatively, the compound of formula [I-(S)] may be optionally converted into a pharmaceutically acceptable salt thereof by reacting it with an acid. Non-limiting examples of pharmaceutically acceptable salts include acid addition salts such as those formed with inorganic acids such as hydrochloric, hydrobromic, sulfuric or phosphoric acids as well as organic acids such as succinic, tartaric, malonic, citric, maleic, acetic, fumaric or methanesulfonic acid.

The preparation of the salts of a compound of formula [I-(R)], or a compound of formula [I-(S)] can be carried out by methods known in the art. Generally, such salts are, for example, prepared by reacting them with a stoichiometric amount of the appropriate pharmaceutically acceptable acid in water or in an organic solvent or in a mixture of them and optionally heating.

As described above, either the compounds of formula [I-(R)] or, alternatively, of formula [I-(S)] can be obtained from the corresponding salts of formula [II-(R,S)] or [II-(S,R)] respectively, which precipitates from the corresponding reaction medium.

Alternatively, either the compounds of formula [I-(R)] or, alternatively, of formula [I-(S)] can be obtained directly from the corresponding salts of formula [II-(R,R)] or [II-(S,S)] respectively, which remain in solution by cleaving the salt into the corresponding enantiomers. This process forms also part of the invention. In one embodiment, the cleavage of the salts is performed by submitting the salts [II-(S,S)] or, alternatively, [II-(R,R)] to either a base treatment, or alternatively,

to first an acid treatment and then a base treatment as described above. After isolation of the enantiomer [I-(R)] or, alternatively, [I-(S)], they may be optionally reacted with an acid to give the corresponding pharmaceutically acceptable salt.

A mentioned above, the enantiomeric mixture of step (a) may contain a compound of formula [I-(R)] and a compound of formula [I-(S)] of step (a) at any relative proportion to one another.

In one embodiment, optionally in combination with one or more features of the various embodiments described above or below, the enantiomeric mixture of step (a) is a racemic mixture.

In another embodiment, optionally in combination with one or more features of the various embodiments described above or below, the enantiomeric mixture of step (a) is enriched in the compound of formula [I-(R)]; the agent used is a compound of formula [III-(S)]; and the obtained diastereomeric mixture contains a compound of formula [II-(R,S)] and a compound of formula [II-(S,S)].

In another embodiment, optionally in combination with one or more features of the various embodiments described above or below, the process of the invention comprises the preparation of an enantiomeric mixture enriched in the compound of formula [I-(R)]. Thus, in one embodiment the process of the invention further comprises the following steps prior to step (a):

(i) reacting a racemic mixture containing a compound of formula [I-(R)] and a compound of formula [I-(S)] with a chiral agent which is a compound of formula [III-(R)] in the presence of a solvent mixture containing water and one or more organic solvents which are miscible with water; to give a diastereomeric mixture containing a compound of formula [II-(S,R)] and a compound of formula [II-(R,R)];
(ii) separating the compound of formula [II-(S,R)] from the diastereomeric mixture of the previous step, whereby the compound of formula [II-(R,R)] remains in solution; and
(iii) submitting the compound of formula [II-(R,R)] to either a base treatment, or alternatively, to first an acid treatment and then a base treatment to give an enantiomeric mixture enriched in the compound of formula [I-(R)].

In another embodiment, optionally in combination with one or more features of the various embodiments described above or below, the enantiomeric mixture of step (a) is enriched in the compound of formula [I-(S)]; the agent used is a compound of formula [III-(R)]; and the obtained diastereomeric mixture contains a compound of formula [II-(S,R)] and a compound of formula [II-(R,R)].

In another embodiment, optionally in combination with one or more features of the various embodiments described above or below, the process of the invention also comprises the preparation of the enantiomeric mixture enriched in the compound of formula [I-(S)]. Thus, in one embodiment the process of the invention before step (a) further comprises the following steps:

(i') reacting a racemic mixture containing a compound of formula [I-(R)] and a compound of formula [I-(S)] with a chiral agent which is a compound of formula [III-(S)] in the presence of a solvent mixture containing water and one or more organic solvents which are miscible with water; to give a diastereomeric mixture containing a compound of formula [II-(R,S)] and a compound of formula [II-(S,S)];
(ii') separating the compound of formula [II-(R,S)] from the diastereomeric mixture of the previous step, whereby the compound of formula [II-(S,S)] remains in solution; and
(iii') submitting the compound of formula [II-(S,S)] to either a base treatment, or alternatively, to first an acid treatment and then a base treatment to give an enantiomeric mixture enriched in the compound of formula [I-(S)].

In the above processes steps (i) and (i') may be carried out under the conditions already explained for step (a). Steps (ii) and (ii') may be carried out under the conditions already explained for step (b); and steps (iii) and (iii') may be carried out under the conditions already explained for step (d). Thus, particular and preferred embodiments previously described for steps (a), (b) and (d) are also particular and preferred embodiments for steps (i)/(i'), (ii)/(ii') and (iii)/(iii') respectively.

The racemic starting compound of formula (I) used in the present invention may be prepared by several processes known in the art such as the ones described in WO 93/00086, WO 94/14772, and WO 99/54302.

Alternatively, the racemic compound of formula (I) may be prepared by the process described in scheme 1:

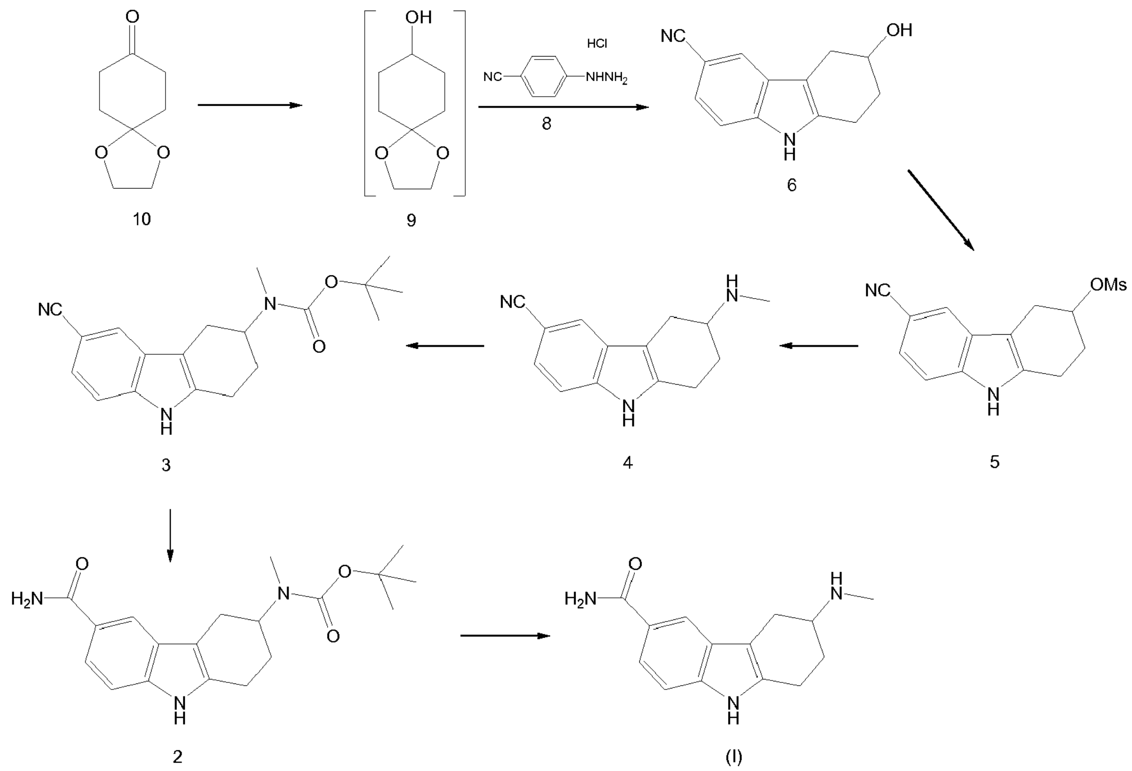


10 9 6 5 4 3 2 (I)

Scheme 1

Starting compound (10) is reduced to give compound (9) with a suitable reducing agent such as $NaBH_4$ in an appropriate solvent, such as methanol. Compound (9) is reacted with the corresponding phenylhydrazine (8) under acidic conditions, such as aqueous HCl, to give compound (6). The hydroxyl group of compound (6) is converted into a leaving group such as a mesylate group (5) for example with mesyl chloride in the presence of a base, such as diisopropylethylamine, in an appropriate solvent. Compound (5) is reacted with methylamine in an appropriate solvent, such as water, to give compound (4), which is subsequently converted to compound (3) with di-tert-butyl carbonate in an appropriate solvent, such as tetrahydrofuran. By reacting compound (3) with an oxidizing agent such as oxygen peroxide in the presence of sodium hydroxide, and subsequent deprotection under acidic conditions such as HCl/methanol, the compound of formula (I) is obtained.

Throughout the description and claims the word "comprise" and variations of the word, are not intended to exclude other technical features, additives, components, or steps. Furthermore, the word "comprise" encompasses the case of "consisting of". Additional objects, advantages and features of the invention will become apparent to those skilled in the art upon examination of the description or may be learned by practice of the invention. The following examples and drawings are provided by way of illustration, and they are not intended to be limiting of the present invention. Reference signs related to drawings and placed in parentheses in a claim, are solely for attempting to increase the intelligibility of the claim, and shall not be construed as limiting the scope of the claim. Furthermore, the present invention covers all possible combinations of particular and preferred embodiments described herein.

EXAMPLES

Nuclear magnetic resonance spectrometry: NMR spectra were obtained using a Varian Mercury Plus 400 MHz spectrometer at 25 °C, using DMSO-$d_6$ as solvent and tetramethylsilane as reference.

Example 1. (R)-(+)-6-carboxamido-3-N-methylamino-1,2,3,4-tetrahydro-carbazole (S)-(3,5-dinitrobenzoyl)phenylglycinate (compound of formula [II-(R,S)]) (starting from racemic mixture)

a) Resolution process

A three-necked flask equipped with a reflux condenser and mechanical stirring was charged with racemic 3-methylamino-6-carboxamido-1,2,3,4-tetrahydrocarbazole (25 g, 0.103 mol), (S)-(3,5-dinitrobenzoyl)phenylglycine (28.38 g; 0.082 mol) followed by dimethylformamide (DMF) (250 mL) and water (500 mL). The mixture was heated at reflux until total dissolution (100 °C). The mixture was cooled to room temperature, followed by stirring at this temperature for 5 h. The solid was collected by filtration, washed with water (3 x 25 mL) and dried in vacuo at a temperature up to 50 °C to give compound of formula [II-(R,S)] as an orange solid (36.25 g, 59.9% yield, 70.0 ee%).

b) First purification

A three-necked flask equipped with a reflux condenser and mechanical stirring was charged with compound of formula [II-(R,S)] (as obtained above) followed by dimethylformamide (362 mL) and water (725 mL). The mixture was heated at reflux until total dissolution (100 °C). The mixture was cooled to room temperature, followed by stirring at this temperature for 3 h. The solid was collected by filtration, washed with water (3 x 25 mL) and dried in vacuo at a temperature up to 50 °C to give compound of formula [II-(R,S)] as an orange solid (25.71 g, 70.9% w/w yield, 42.5% global, 98.0 ee%).

c) Second purification

A three-necked flask equipped with a reflux condenser and mechanical stirring was charged with compound of formula [II-(R,S)] (as obtained above) followed by dimethylformamide (257 mL) and water (515 mL). The mixture was heated at reflux until total dissolution (100 °C). The mixture was cooled to room temperature, followed by stirring at this temperature for 3 h. The solid was collected by filtration, washed with water (3 x 25 mL) and dried in vacuo at a temperature up to 50 °C to give compound of formula [II-(R,S)] as an orange solid (23.61 g, 91.8% w/w yield, 39.0% global yield, 99.3 ee%).

$^1$H-RMN Chemical shift (ppm): 1.89-1.83 m (1 H); 2.22 br (1 H); 2.60 s (3H); 2.77-2.67 m (4H); 3.11 dd (1 H); 3.31 br (1 H); 5.31 d (1 H); 7.03 br (1 H); 7.28-7.17 m (4H); 7.48 d (1 H); 7.57 dd (1 H); 7.77 br (1 H); 7.93 s (1 H); 8.85 t (1 H); 9.01 d (2H); 9.50 d (1 H); 11.07 s (1 H).

$^{13}$C-RMN Chemical shift (ppm): 20.87 (CH2); 23.90 (CH2); 25.41 (CH2); 30.10 (CH3); 54.45 (CH); 59.40 (CH); 105.97 (C q); 109.95 (CH); 117.48 (CH); 120.40 (CH); 120.45 (CH); 124.56 (C q); 126.18 (C q); 126.44 (CH); 127.49 (CH); 127.64 (CH); 127.73 (CH); 134.68 (C q); 137.09 (C q); 137.82 (C q); 140.91 (C q); 147.87 (C q); 161.12 (C q); 169.00 (C q); 171.83 (C q).

Example 2. Compound of formula [II-(R,S)] (starting from racemic mixture)

Following the same process as described in example 1 a) but starting from 5 g of racemic compound of formula (I) and using dimethylacetamide instead of dimethylformamide, the compound of formula [II-(R,S)] was obtained in an enantiomeric excess of 48.6%. After purification by recrystallization as described in example 1 b) but using dimethylacetamide instead of dimethylformamide, the compound of formula [II-(R,S)] was obtained in 43.8% yield and 93.0 ee%.

Examples 3-8. Compound of formula [II-(R,S)] (starting from racemic mixture)

Following an analogous process to the one described in example 1 a) but using the conditions indicated in table 1, compound of formula [II-(R,S)] was obtained.

Table 1

| Example | Solvent mixture (ratio v:v) | Volumes of solvent[a)] | mol [III-(S)]/ mol (I) | Yield (%) | ee (%) |
|---|---|---|---|---|---|
| 3 | DMF:water 1:1 | 20 | 0.5 | 25.3 | 87.5 |
| 4 | ACN:water 7:3 | 25 | 0.5 | 27.4 | 91.5 |
| 5 | THF:water 7:3 | 10 | 0.5 | 20.7 | 86.7 |
| 6 | iBuOH:water 7:3 | 20 | 0.5 | 38.9 | 75.8 |
| 7 | iPrOH:water 7:3 | 20 | 1 | 53.9 | 46.3 |
| 8 | EtOH:water 1:1 | 20 | 1 | 53.9 | 71.5 |
| [a)] Volumes of solvent per g of compound of formula (I) | | | | | |

Example 9. Compound of formula [II-(R,S)] (starting from a mixture enantiomerically enriched)

A three-necked flask equipped with a reflux condenser and mechanical stirring was charged with racemic 3-methylamino-6-carboxamido-1,2,3,4-tetrahydrocarbazole (4.5 g, 0.018 mol), (R)-(3,5-dinitrobenzoyl)phenylglycine (4.79 g, 0.014 mol) followed by dimethylformamide (45 mL) and water (90 mL). The mixture was heated at reflux until total dissolution (100 °C). The mixture was cooled to room temperature, followed by stirring at this temperature for 4 h. The precipitated solid was separated by filtration and washed with water (3 x 5 mL). The resulting mother liquors were added to ethyl acetate (45 mL) and the pH was adjusted below 1.0 using HCl. From the resulting biphasic solution the organic phase (FO1) and the aqueous phase (FA1) were separated. The FO1 was washed with HCl 0.5 N (9 mL) and the aqueous phase (FA2) was collected and mixed with FA1.

The pH of the mixed aqueous phases was adjusted between 10.0 and 11.0 using NaOH solution 50.0%. The resulting mixture was extracted twice with isobutanol (45 mL) and the resulting organic phases were concentrated to vacuum.

To the resulting residue (S)-(3,5-dinitrobenzoyl)phenylglycine (4.79 g, 0.014 mol) followed by dimethylformamide (40 mL) and water (80 mL) were added. The mixture was heated at reflux until total dissolution (100 °C). The mixture was cooled to room temperature, followed by stirring at this temperature for 4 h. The solid was collected by filtration washed with water (3 x 5 mL) and dried in vacuo at a temperature up to 50 °C to give compound of formula [II-(R,S)] as an orange solid (4.90 g, 45.0% yield, 96.2 ee%).

Example 10. (R)-(+)-6-Carboxamido-3-N-methylamino-1,2,3,4-tetrahydrocarbazole succinate monohydrate (succinic salt of (compound of formula [I-(R)])

A flask equipped with a mechanical stirrer was charged with (R)-(+)-6-carboxamido-3-N-methylamino-1,2,3,4-tetrahydrocarbazole (S)-(3,5-dinitrobenzoyl)phenylglycinate (30 g, 0.05 mol), water (600 mL) and ethyl acetate (300 mL).

The mixture was acidified to pH 0.5 - 1.0 until total dissolution using concentrated hydrochloric acid. The phases were separated and the aqueous layer was washed with ethyl acetate (300 mL), adjusted to pH 10.5 - 12 using 50% aqueous sodium hydroxide and extracted with isobutanol (2 x 375 mL). The combined isobutanol extracts were washed with water (60 mL) and concentrated to a volume of about 60 mL by rotary evaporation, at which point ethanol (210 mL), water (30 mL) and succinic acid (6.02g, 0.05 mol) were added.

The mixture was heated at reflux until total dissolution and then it was cooled to room temperature, followed by stirring at this temperature overnight. The solid was collected by filtration, washed with ethanol (3 x 10 mL) and dried in vacuo at a temperature up to 40 °C to give (R)-(+)-6-carboxamido-3-N-methylamino-1,2,3,4-tetrahydrocarbazole succinate monohydrate, a white solid (16.58 g, 85.7% yield).

Example 11. (S)-(+)-6-Carboxamido-3-N-methylamino-1,2,3,4-tetrahydrocarbazole (R)-(3,5-dinitrobenzoyl)phenylglycinate (compound of formula [II-(S,R)]) (starting from racemic mixture)

a) Resolution process

A three-necked flask equipped with a reflux condenser and mechanical stirring was charged with racemic 3-methylamino-6-carboxamido-1,2,3,4-tetrahydrocarbazole (4.5 g, 0.018 mol), (R)-(3,5-dinitrobenzoyl)phenylglycine (R-DNBG)(4.79 g; 0.014 mol) followed by dimethylformamide (45 mL) and water (90 mL). The mixture was heated at reflux until total dissolution (100 °C). The mixture was cooled to room temperature, followed by stirring at this temperature for 4 h. The solid was collected by filtration, washed with water (3 x 5 mL) and dried in vacuo at a temperature up to 50 °C to give compound of formula [II-(S,R)] as an orange solid (5.27 g, 48.4% yield, 68.0 ee%).

b) First purification

A three-necked flask equipped with a reflux condenser and mechanical stirring was charged with compound of formula [II-(S,R)] (as obtained above) followed by dimethylformamide (53 mL) and water (106 mL). The mixture was heated at reflux until total dissolution (100 °C). The mixture was cooled to room temperature, followed by stirring at this temperature for 3 h. The solid was collected by filtration, washed with water (3 x 5 mL) and dried in vacuo at a temperature up to 50° C to give compound of formula [II-(S,R)] as an orange solid (4.28 g, 81.2% w/w yield, 39.3% global yield, 98.0 ee%).

c) Second purification

A three-necked flask equipped with a reflux condenser and mechanical stirring was charged with compound

of formula [II-(S,R)] (as obtained above) followed by dimethylformamide (43 mL) and water (86 mL). The mixture was heated at reflux until total dissolution (100 °C). The mixture was cooled to room temperature, followed by stirring at this temperature for 3h. The solid was collected by filtration, washed with water (3 x 5 mL) and dried in vacuo at a temperature up to 50 °C to give compound of formula [II-(S,R)] as an orange solid (4.10 g, 96.0% w/w yield, 37.8% global yield, 99.8 ee%).

$^{1}$H-RMN Chemical shift (ppm): 1.89-1.84 m (1 H); 2.22 br (1 H); 2.60 s (3H); 2.77-2.64 m (4H); 3.10 dd (1 H); 3.31 br (1 H); 5.31 d (1 H); 7.02 br (1 H); 7.28-7.16 m (4H); 7.48 d (1 H); 7.57 dd (1 H); 7.76 br (1 H); 7.93 s (1 H); 8.85 t (1 H); 9.01 d (2H); 9.49 d (1 H); 11.06 s (1 H).

$^{13}$C-RMN Chemical shift (ppm): 20.87 (CH2); 23.98 (CH2); 25.43 (CH2); 30.13 (CH3); 54.46 (CH); 59.40 (CH); 105.98 (C q); 109.94 (CH); 117.48 (CH); 120.40 (CH); 120.46 (CH); 124.56 (C q); 126.18 (C q); 126.44 (CH); 127.48 (CH); 127.65 (CH); 127.73 (CH); 134.68 (C q); 137.09 (C q); 137.82 (C q); 140.91 (C q); 147.87 (C q); 161.12 (C q); 169.00 (C q); 171.78 (C q);

Example 12. Compound of formula [II-(S,R)] (starting from racemic mixture)

Following an analogous process as described in example 11 a) but using as a solvent mixture 39 volumes of solvent consisting of a mixture of acetonitril and water in a ratio 9:1 v:v; and 0.5 equivalents of R-DNBG, the compound of formula [II-(S,R)] was obtained in 41.0% yield and 75.0 ee%.

Comparative example. Resolution process of (compound of formula (I) with other chiral agents

Following an analogous process to the one described in example 1 a) but using the conditions indicated in table 2, compound of formula [II-(R,S)] (designated as R) or compound of formula [II-(S,R)] (designated as S) was obtained.

Table 2

| Comparative example | Chiral agent | Solvent mixture (ratio v:v) | Volumes of solvent[c)] | mol [III-(S)]/ mol (I) | ep[d)] (%) | ee (%) |
|---|---|---|---|---|---|---|
| Comp. ex.1 | L-(+)-tartaric acid | MeOH:water 9:1 | 25 | 0.5 | 52 (S) | 4 |
| Comp. ex.2 | L-(-)-tartaric acid | MeOH:water 9:1 | 25 | 0.5 | 53 (R) | 6 |
| Comp. ex.3 | (1R)-(-)-Camphorsulfonic acid | EtOH | 5 | 1 | 50 (R) | 0 |
| Comp. ex.4 | (1S)-(+)-Camphorsulfonic acid [a)] | EtOH | 5 | 1 | 52 (S) | 4 |
| Comp. ex.5 | di-p-toluoyl-(D)-(+)-tartaric acid (DPTTA) [b)] | iBuOH | 12.5 | 0.5 | 50 (R) | 0 |
| Comp. ex.6 | L-Pyroglutamic acid | EtOH:water 12,5:1 | 5.4 | 1 | 50 (R) | 0 |
| Comp. ex.7 | L-Pyroglutamic acid | iPrOH:water 20:5 | 20 | 1 | 51 (R) | 2 |

[a)] chiral agent disclosed in WO 94/14772
[b)] chiral agent disclosed in WO 2012/14020
[c)] Volumes of solvent per g of compound of formula (I)
[d)] enantiomeric purity with respect to the major enantiomer

REFERENCES CITED IN THE APPLICATION

- WO 94/14772

- WO 2012/147020

## Claims

**1.** A process for the preparation of either a compound of formula [II-(R,S)] or, alternatively, a compound of formula [II-(S,R)]

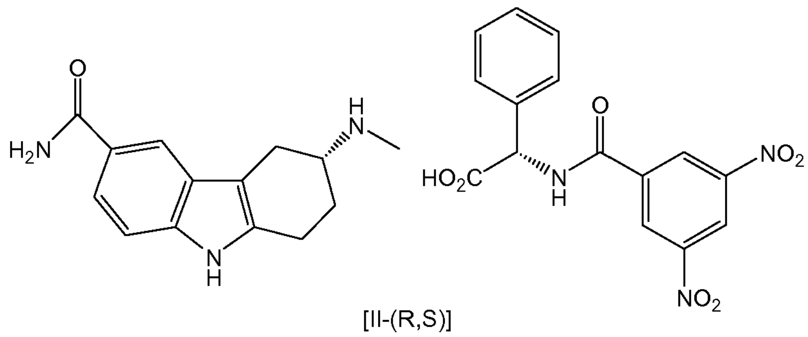

[II-(R,S)]

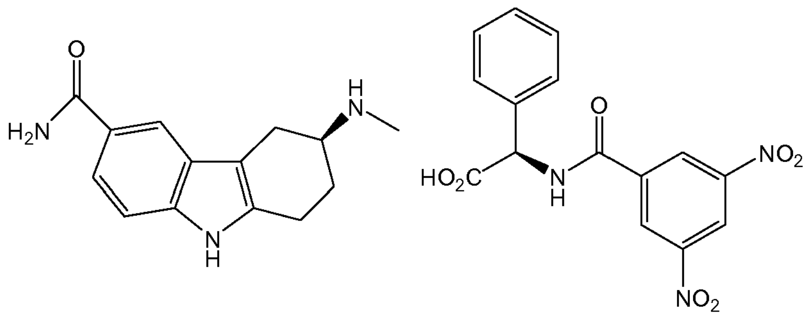

[II-(S,R)]

which comprises the following steps:

(a) reacting an enantiomeric mixture formed by the two enantiomers of 6-carboxamido-3-N-methylamino-1,2,3,4-tetrahydrocarbazole (I), which are the compound of formula [I-(R)] and the compound of formula [I-(S)]

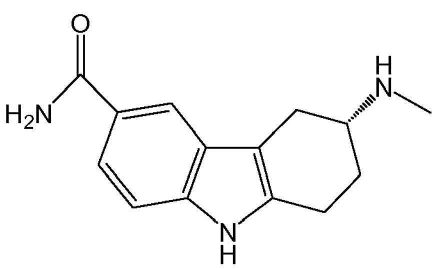

[I-(R)]

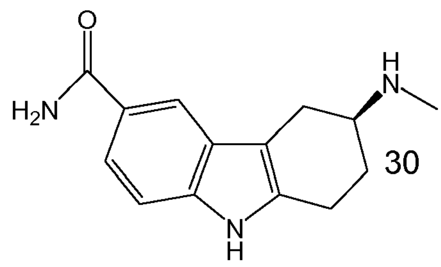

[I-(S)]

with a chiral agent selected from the two enantiomers of (3,5-dinitrobenzoyl)-phenylglycine (III) which are the compound of formula [III-(S)] and the compound of formula [III-(R)]

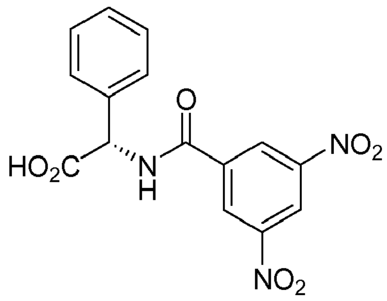

[III-(S)]

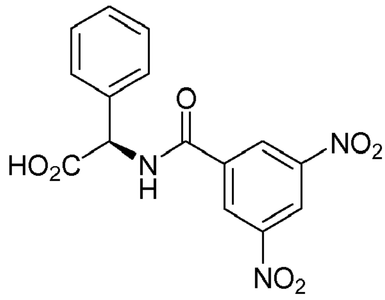

[III-(R)]

in the presence of a solvent mixture containing water and one or more organic solvents which are miscible with water; to give a diastereomeric mixture formed by one of the enantiomers of the compound of formula (I) with one of the enantiomers of the compound of formula (III), the diastereomeric mixture containing either

A) a compound of formula [II-(R,S)] and a compound of formula [II-(S,S)] when the chiral agent used is a compound of formula [III-(S)], or alternatively,
B) a compound of formula [II-(S,R)] and a compound of formula [II-(R,R)] when the chiral agent used is a compound of formula [III-(R)];

(b) isolating either the compound of formula [II-(R,S)] or, alternatively, the compound of formula [II-(S,R)] from the corresponding diastereomeric mixture of step (a) whereby either the compound of formula [II-(S,S)], or alternatively, the compound of formula [II-(R,R)] remains in solution; and
(c) optionally purifying either the compound of formula [II-(R,S)] or, alternatively, the compound of formula [II-(S,R)] obtained in step (b).

**2.** The process according to claim 1, further comprising the following steps:

(d) submitting either the compound of formula [II-(R,S)] or, alternatively, the compound of formula [II-(S,R)] obtained in step (c) to either a base treatment, or alternatively, to first an acid treatment and then a base treatment to give a compound of formula [I-(R)], or a compound of formula [I-(S)], respectively;
(e) isolating either the compound of formula [I-(R)] or, alternatively, the compound of formula [I-(S)] from the corresponding reaction medium of step (d); and
(f) optionally reacting either the compound of formula [I-(R)], or, alternatively, the compound of formula [I-(S)] obtained in step (d) with an acid to give the corresponding pharmaceutically acceptable salt.

**3.** The process according to any of the claims 1-2, for the preparation of a compound of formula [II-(R,S)], wherein the chiral agent is a compound of formula [III-(S)].

**4.** The process according to any of the claims 1-3, wherein the enantiomeric mixture of step (a) is a racemic mixture.

**5.** The process according to any of the claims 1-3, wherein in step (a) the enantiomeric mixture is enriched in the compound of formula [I-(R)]; the chiral agent used is a compound of formula [III-(S)]; and the obtained diastereomeric mixture contains a compound of formula [II-(R,S)] and a compound of formula [II-(S,S)].

**6.** The process according to claim 5, further comprising the following steps prior to step (a):

(i) reacting a racemic mixture containing a compound of formula [I-(R)] and a compound of formula [I-(S)] with a chiral agent which is a compound of formula [III-(R)] in the presence of a solvent mixture containing water and one or more organic solvents which are miscible with water; to give a diastereomeric mixture containing a compound of formula [II-(S,R)] and a compound of formula [II-(R,R)];
(ii) separating the compound of formula [II-(S,R)] from the diastereomeric mixture of the previous step, whereby the compound of formula [II-(R,R)] remains in solution; and
(iii) submitting the compound of formula [II-(R,R)] to either a base treatment, or alternatively, to first an acid treatment and then a base treatment to give an enantiomeric mixture enriched in the compound of formula [I-(R)].

**7.** The process according to any of the claims 1-6, wherein the solvent mixture used for reacting the mixture containing [I-(R)] and [I-(S)] with the chiral agent is a mixture of water and one or more organic solvents selected from the group consisting of methanol, ethanol, propanol, isopropanol, acetone, acetonitrile, tetrahydrofuran, dimethylformamide, dimethylsulfoxide, dimethylacetamide, and mixtures thereof.

**8.** The process according to claim 7, wherein the organic solvent is selected from the group consisting of dimethylformamide, dimethylsulfoxide, dimethylacetamide, and mixtures thereof.

**9.** The process according to any of the claims 1-8, wherein the molar ratio of the mixture containing [I-(R)] and [I-(S)] and the chiral agent is from 0.4:1 to 1.6:1.

**10.** The process according to any of the claims 1-9, wherein the reaction of the mixture containing [I-(R)] and [I-(S)] with the chiral agent is carried out at a temperature comprised from room temperature to the reflux temperature of the solvent mixture and the process further comprises the step of cooling the reaction medium to a temperature comprised from 0-25 °C.

**11.** The process according to any of the claims 1-10, wherein the isolation or separation of the compound of formula [II-(R,S)] or, alternatively, the compound of formula [II-(S,R)] from the corresponding diastereomeric mixture is carried out by filtration.

**12.** The process according to any of the claims 1-11, wherein step (c) comprises one or more recrystallization steps in a suitable solvent or solvent mixture.

**13.** The process according to claim 12, wherein the solvent mixture for the recrystallization steps is the same as the one used for the reaction of the mixture containing [I-(R)] and [I-(S)] with the chiral agent.

**14.** A compound of formula [II-(R,S)].

**15.** A compound of formula [II-(S,R)].

Europäisches Patentamt
European Patent Office
Office européen des brevets

# EUROPEAN SEARCH REPORT

Application Number
EP 13 38 2233

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | WO 94/14772 A1 (SMITHKLINE BEECHAM PLC [GB]; BORRETT GARY THOMAS [GB]; KITTERINGHAM JO) 7 July 1994 (1994-07-07)<br>* examples 3,13 *<br>----- | 1-15 | INV.<br>C07D209/88 |
| A,D | WO 2012/147020 A1 (ORCHID CHEMICALS AND PHARAMCEUTICALS LTD [IN]; REGURI BUCHI REDDY [IN]) 1 November 2012 (2012-11-01)<br>* Page 8, lines14-23; page 9, lines 19-23 *<br>----- | 1-15 | |
| A | WO 2010/043571 A1 (INTERQUIM SA [ES]; MARQUILLAS OLONDRIZ FRANCISCO [ES]; POMARES MARCO M)<br>22 April 2010 (2010-04-22)<br>* claim 1 *<br>----- | 1-15 | |
| | | | TECHNICAL FIELDS SEARCHED (IPC) |
| | | | C07D |

The present search report has been drawn up for all claims

1

| Place of search | Date of completion of the search | Examiner |
|---|---|---|
| Munich | 30 October 2013 | Cooper, Simon |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
.......................................................................
& : member of the same patent family, corresponding document

EPO FORM 1503 03.82 (P04C01)

## ANNEX TO THE EUROPEAN SEARCH REPORT ON EUROPEAN PATENT APPLICATION NO. EP 13 38 2233

This annex lists the patent family members relating to the patent documents cited in the above-mentioned European search report. The members are as contained in the European Patent Office EDP file on
The European Patent Office is in no way liable for these particulars which are merely given for the purpose of information.

30-10-2013

| Patent document cited in search report | Publication date | Patent family member(s) | Publication date |
|---|---|---|---|
| WO 9414772 A1 | 07-07-1994 | AT 233239 T | 15-03-2003 |
| | | AU 688748 B2 | 19-03-1998 |
| | | AU 2851797 A | 16-10-1997 |
| | | CA 2152630 A1 | 07-07-1994 |
| | | CN 1095712 A | 30-11-1994 |
| | | DE 69332718 D1 | 03-04-2003 |
| | | DE 69332718 T2 | 04-12-2003 |
| | | DK 0674621 T3 | 10-06-2003 |
| | | EP 0674621 A1 | 04-10-1995 |
| | | ES 2193151 T3 | 01-11-2003 |
| | | HK 1012368 A1 | 26-11-2004 |
| | | JP 3364222 B2 | 08-01-2003 |
| | | JP H08504790 A | 21-05-1996 |
| | | NZ 259363 A | 20-12-1996 |
| | | PT 674621 E | 31-07-2003 |
| | | US 5618947 A | 08-04-1997 |
| | | US 5650426 A | 22-07-1997 |
| | | WO 9414772 A1 | 07-07-1994 |
| | | ZA 9309457 A | 19-06-1995 |
| WO 2012147020 A1 | 01-11-2012 | NONE | |
| WO 2010043571 A1 | 22-04-2010 | AU 2009305477 A1 | 22-04-2010 |
| | | CA 2735101 A1 | 22-04-2010 |
| | | CN 102143937 A | 03-08-2011 |
| | | EP 2346811 A1 | 27-07-2011 |
| | | ES 2396066 T3 | 19-02-2013 |
| | | JP 2012505183 A | 01-03-2012 |
| | | KR 20110069036 A | 22-06-2011 |
| | | PE 03662011 A1 | 20-06-2011 |
| | | RU 2011119224 A | 20-11-2012 |
| | | US 2011152543 A1 | 23-06-2011 |
| | | WO 2010043571 A1 | 22-04-2010 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9300086 A
- WO 9414772 A
- WO 2012147020 A
- WO 9954302 A
- WO 201214020 A